## Europäisches Patentamt

## European Patent Office

## Office européen des brevets

(11) Veröffentlichungsnummer: **0 410 372 A1**

## (12) EUROPÄISCHE PATENTANMELDUNG

(21) Anmeldenummer: **90114150.7**

(22) Anmeldetag: **24.07.90**

(51) Int. Cl.5: **C07K 5/08**, C07K 5/10, C07K 7/06, C07K 5/02, A61K 37/02

(30) Priorität: **27.07.89 DD 331201**

(43) Veröffentlichungstag der Anmeldung:
**30.01.91 Patentblatt 91/05**

(84) Benannte Vertragsstaaten:
**AT BE CH DE ES FR GB IT LI NL SE**

(71) Anmelder: **BERLIN-CHEMIE AG**
**Glienicker Weg 125-127**
**O-1199 Berlin(DD)**

(72) Erfinder: **Forner, Klaus, Dr.**
**Florastrasse 34**
**O-1100 Berlin(DE)**
Erfinder: **Diezel, Wolfgang, Prof.Dr.sc.med.**
**Anton-Saefkow-Platz 3**
**O-1156 Berlin(DE)**
Erfinder: **Georgi, Monika**
**Frankfurter Allee 178**
**O-1130 Berlin(DE)**
Erfinder: **Ehrlich, Angelika**
**Pekrunstrasse 69**
**O-1140 Berlin(DE)**
Erfinder: **Weber, Harald**
**Stargarder Strasse 48**
**O-1058 Berlin(DE)**
Erfinder: **Simon Hans-Ullrich, Dr.med.**
**Hugo-Schrader-Strasse 45**
**O-6908 Jena(DE)**

(74) Vertreter: **Hansen, Bernd, Dr. Dipl.-Chem. et al**
**Hoffmann, Eitle & Partner Patent- und**
**Rechtsanwälte Arabellastrasse 4e 4**
**D-8000 München 81(DE)**

(54) **Verfahren zur Herstellung von humanen Spleninderivaten.**

(57) Humanes Splenin, humane Splenotritine, humane Splenopentine, acylierte Splenotritine, acylierte Splenopentine, Acetylierung, Immunbiologie, Immundefizienzen, Immunsystem, Autoimmunreaktionen, Knochenkark-Stammzellen, AIDS, Pharmazie

Die Erfindung betrifft humane Spleninderivate der Formel I und Verfahren zu deren Herstellung. Die nach üblichen Synthesemethoden der Peptidchemie hergestellten neuen humanen Spleninderivate weisen eine hohe immunmodulierende Aktivität und hohe Stabilität auf, wobei sie keine neurotropen Nebenwirkungen zeigen. Die erfindungsgemäßen Verbindungen können zur Normalisierung der Funktion des Immunsystems bei primären und sekundären Immundefizienzen und zur Stimulierung von Stammzellen des Knochenmarks eingesetzt werden.

## VERFAHREN ZUR HERSTELLUNG VON HUMANEN SPLENINDERIVATEN

Anwendungsgebiet der Erfindung

Die Erfindung betrifft Verfahren zur Herstellung von humanen Spleninderivaten der Formel I. Diese Verbindungen weisen ausgezeichnete immunbiologische Eigenschaften auf. Sie besitzen eine immunmodulierende Aktivität und üben u.a. einen direkten Einfluß auf die Proliferation und Differenzierung von Stammzellen des Knochenmarks aus.
Anwendungsgebiete der Erfindung sind die Medizin und die pharmazeutische Industrie.

Charakteristik der bekannten technischen Lösungen

Für die Immunabwehr des Organismus ist das abgestimmte Zusammenspiel aller Blutzellen von entscheidender Bedeutung. Die Proliferation und Differenzierung (Funktionalisierung) der Blutzellen wird durch Hormone beeinflußt, die durch primäre und sekundäre Lymphorgane, wie Thymus und Milz, sekretiert werden. So wird durch das 1981 erstmals beschriebene Rinder-Thymopoietin (bTP) die Differenzierung von T-Lymphozyten stimuliert und die von B-Lymphozyten inhibiert, sowie die neuromuskuläre Transmission beeinflußt, während durch das Rinder-Splenin (bSP) die Differenzierung sowohl von T- und B-Lymphozyten stimuliert wird und keine Wirkung auf die neuromuskuläre Transmission erfolgt (AUDHYA et al., Biochemistry 1981 , (20), 6195).
1987 wurden die aus menschlichem Thymus und Milz isolierten Hormone hTP und hSP beschrieben (AUDHYA et al., Proc. Nat. Acad. Sci., USA, 1987 , (84), 3345).
Die biologischen Wirkungen dieser aus 49 bzw. 48 Aminosäuren aufgebauten Hormone werden durch deren sog. "aktive Zentren", dem Fragment 32-36, reproduziert.
Diese als Thymo- bzw. Splenopentine bezeichneten Pentapeptide weisen die Struktur Arg Lys X Val Tyr auf, wobei im Falle des bTP5 und hTP5 X = Asp ist, während für bSP5 X = Glu und für hSP5 X = Ala ist.
Gegenwärtig wird hTP5 als "Timunox" zur Behandlung von sekundären Immundefizienzen eingesetzt.

Ziel der Erfindung

Das Ziel der Erfindung besteht darin, der pharmazeutischen Industrie und Medizin neue immunbiologisch wirksame Verbindungen zur Verfügung zu stellen.

Darlegung des Wesens der Erfindung

Der Erfindung liegt die Aufgabe zugrunde, neue Peptide, die einerseits eine höhere immunmodulierende Aktivität und höhere Stabilität und andererseits keine neurotropen Nebenwirkungen aufweisen und einfach herstellbar sind, zu finden sowie Verfahren zu deren Herstellung bereitzustellen.
Die Aufgabe wird durch ein Verfahren zur Herstellung von humanen Spleninderivaten der Formel I
Arg - X - Ala - $R^1$     I,
worin
Arg ggf. substituiertes Arginin,
X Sar, Pro, Gly, Ala, Leu, Ile, Thr, Ser, Lys, Val, Phe oder Cys, die ggf. substituiert sind,
$R^1$ -O $R^3$, -N ($R^3$ $R^4$), -X-Z-O $R^3$ oder -X-Z-N ($R^3$ $R^4$),
worin
$R^3$ und $R^4$ gleich oder verschieden sind und H, $C_1$-$C_{18}$-Alkyl, $C_2$-$C_7$ - Alkenyl , $C_2$-$C_7$ - Alkinyl, $C_7$-$C_{20}$ - Alkaryl, $C_7$-$C_2$, - Aralkyl oder Polyhydroxyalkyl
und
Z Tyr, Phe, decarboxy-Tyr, decarboxy-Phe , die ggf. durch OH, $NO_2$, Halogen und $OCH_3$ substituiert sind, oder Trp
bedeuten,
die Aminosäuren jeweils die L - oder D - Konfiguration aufweisen können,
sowie deren durch Verknüpfung der α- bzw. ε-Aminogruppe mit der α-Carboxylgruppe des Alanins bzw. der Aminosäure Z direkt oder mit Dicarbonsäuren

und/oder durch Verknüpfung der - Carboxylgruppe des Alanins bzw. der Aminosäure Z über Polyoxyethylen-, Polyoxyethylendiamino- oder Polymethylendiamino-Ketten

und/oder durch Verknüpfung über S - S - Brückenbindung am Cystein erhaltene Dimere,

sowie deren durch Verknüpfung der Carboxylgruppe der Aminosäure Z mit der Aminogruppe des Arginins erhaltenen cyclischen Derivate,

sowie deren Salze, Komplexe, Amide, Ester und entsprechenden, ggf. partiell, geschützten Derivate gelöst.

Die Herstellung der Spleninderivate der Formel I erfolgt dadurch, daß man die entsprechenden, ggf. geschützten und/oder acylierten Aminosäuren bzw. deren Salze, Komplexe, Amide oder Ester stufenweise oder durch Fragmentkondensation nach an sich bekannten Methoden der Peptidsynthese zu den ggf. geschützten Spleninderivaten der Formel I bzw. deren Salzen, Komplexen, Amiden und Estern umsetzt, die ggf. geschützten freien Spleninderivate der Formel I in ihre Salze, Komplexe, Amide oder Ester überführt und/oder aus den Salzen, Komplexen, Amiden und Estern das freie Peptid der Formel I freisetzt.

Die einzelnen Aminosäuren bzw. Peptidfragmente sowie das Spleninderivat der Formel I können an entsprechender Stelle der Synthese in an sich bekannter Weise acyliert, cyclisiert bzw. dimerisiert werden.

Ebenso können die Schutzgruppen in an sich bekannter Weise ggf. selektiv abgespalten werden.

Die Acylierung der ggf. geschützten Spleninderivate der Formel I und der entsprechenden, ggf. geschützten Aminosäuren bzw. der entsprechenden, ggf. geschützten Di-, Tri- und Tetrapeptidfragmente wird bevorzugt mit N-Hydroxy - norborn - 5- en- 2,3 - dicarboximidestern von Carbonsäuren, insbesondere mit N-Acetoxynorborn - 5-en-2,3-dicarboximid oder mit N-Acetoxybenzotriazol durchgeführt.

Diese Acylierungsmittel sind wegen ihrer geringen Neigung zu unerwünschten Nebenreaktionen und der damit zu erreichenden hohen Ausbeute an Spleninderivaten besonders geeignet.

Aufgrund der hohen Hydrolysebeständigkeit des N-Acetoxynorborn- 5 -en-2,3-dicarboximides besitzt es eine sehr gute Lagerstabilität und kann bei Acylierungsreaktionen sowohl in organischen als auch in wässrigen Medien eingesetzt werden. Außerdem weist es eine hohe Reaktionsfähigkeit mit Aminogruppen auch ohne Basenzusatz auf.

Hinsichtlich ihrer immunmodulierenden Aktivität und ihrer Stabilität sind Verbindungen der Formel I besonders bevorzugt, worin die Aminogruppe des Arginins und/oder der Aminosäure X, insbesondere des Lysins, substituiert ist durch eine der folgenden Gruppierungen:

$C_1$-$C_7$ - Alkyl, $C_6$-$C_{12}$ - Aryl, $C_7$-$C_{20}$ - Alkaryl, $C_7$-$C_{20}$ -Aralkyl, $C_2$-$C_7$ - Alkenyl, $C_2$-$C_7$ - Alkinyl, $C_3$-$C_7$ - Cycloalkyl, $C_3$-$C_7$ - Cycloalkenyl, $C_1$-$C_{18}$ - Alkanoyl, $C_4$-$C_{12}$ -Cycloalkanoyl, $C_7$-$C_{11}$ - Aroyl, $C_6$-$C_{12}$ - Polydroxyalkanoyl, $C_7$-$C_{24}$ - Aralkanoyl,

- $CO(CH_2)_m$ -CO-$R^5$, - $CO(CHOH)_n$-CO-$R^5$, mit m = 1-8 und n = 1-12,

wobei $R^5$ OH bzw. deren entsprecnehde Salze, Ester, und Amide, $N^\alpha$ - Arg - X - Ala - $R^1$ oder $N^\epsilon$ - Lys - (Arg) - Ala - $R^1$ bedeutet.

Unter den Dimeren der Spleninderivate der Formel I weisen v.a. die Verbindungen, die über eine der folgenden Gruppierungen verknüpft sind, eine hohe immunmodulierende Aktivität auf:

-O-$(CH_2)_p$-O-,

-NH-$(CH_2)_p$-NH-,

-O-$(CH_2$-$CH_2$-O-$)_q$-,

-NH-$(CH_2$-$CH_2$-O-$)_q$-$CH_2$-$CH_2$-NH- mit p = 1-18 und q = 1-10 000.

Es wurde gefunden, daß durch $N^\alpha$ - und /oder $N^\epsilon$ -Acylierung, insbesondere Acetylierung, sowohl der Splenotritine als auch der Splenopentine eine erhebliche Steigerung der immunmodulierenden Aktivität erreicht wird, die auf eine deutliche Erhöhung der Abbaustabilität gegenüber Aminopeptidasen und damit verbundene verbesserte Bioverfügbarkeit zurückzuführen ist. Besonders ausgeprägt ist diese Wirkungssteigerung bei Verbindungen der Formel I, worin die $N^\alpha$-Position des Arginins und/oder die $N^\epsilon$-Position des Lysins acyliert, insbesondere acetyliert, ist.

Verbindungen der Formel I, worin X Ala, Ile, Lys, Sar und Pro bedeuten, weisen eine besonders hohe Stabilität auf.

Eine besonders hohe immunmodulierende Aktivität und Stabilität ohne neurotrope Nebenwirkungen zeigen Spleninderivate der Formel I, worin X Lys und $R^1$ Val-Tyr-OR$^3$ oder Val-Tyr-N ($R^3$ $R^4$) bedeuten und Arg und/ oder Lys und/oder Val-Tyr substituiert sind, insbesondere folgende Verbindungen:

($N^\alpha$-Acyl-Arg) - X - Ala - $R^1$,

Arg - ($N^\epsilon$-Acyl-Lys) - Ala-$R^1$,

$N^\alpha$-Acyl-Arg) - ($N^\epsilon$-Acyl- Lys) - Ala - $R^1$ ,

wobei Acyl bevorzugt Acetyl, X bevorzugt Lys und $R^1$ bevorzugt Val-Tyr-OR$^3$ oder Val-Tyr-N ($R^3$ $R^4$) bedeuten, Valin und Tyrosin ggf. substituiert sind.

Die Herstellung von ($N^\alpha$-Acyl-Arg) - ($N^\epsilon$-Acyl- Lys) -Ala-$R^1$ kann durch Acylierung von Arg-Lys-Ala-$R^1$

erfolgen.

Arg -($N^{\epsilon}$-Acyl- Lys) - Ala - $R^1$ und ($N^{\alpha}$-Acyl-Arg) - Lys - Ala - $R^1$ werden durch Acylierung von partiell geschützten Arg-Lys-Ala-R' und anschließende Abspaltung der $N^{\alpha}$ - bzw. $N^{\epsilon}$ - Schutzgruppe hergestellt.

Überraschenderweise zeigen die Splenotritinderivate der Formel I die gleiche hohe immunmodulierende Aktivität und hohe Stabilität wie die Splenopentinderivate der Formel I.

Durch Dimerisierung der Spleninderivate der Formel I bzw. der entsprechenden Fragmente zur Herstellung von dimerisierten Spleninderivaten kann eine weitere Steigerung der immunbiologischen Aktivität erreicht werden.

Von besonderer Bedeutung hinsichtlich der immunbiologischen Aktivität der erfindungsgemäßen Peptide erscheint ihr Effekt der Wirkungssteigerung des GM-CSF (Granulocyten-Macrophagen-Colony stimulating factor), wodurch die Proliferation und Differenzierung von Knochenmarkstammzellen erhöht wird (Tabelle 1).

Offensichlich initiieren die erfindungsgemäß hergestellten Spleninderivate gleichartige biologische Effekte wie der gentechnisch hergestellte GM-CSF, weshalb die Spleninderivate bei solchen Krankheiten Therapiewirkungen zeigen, bei denen auch der GM-CSF therapiewirksam ist: Bei AIDS in Kombination mit Azidothymidin, nach Chemotherapie und autologer Knochenmarktransplantation und bei HIV - Infizierten zwecks Verhinderung des Schweregrades der Erkrankung.

Die Beeinflussung der Bildung bestimmter Typen von Blutzellen ermöglicht sowohl den Bedarf an Bluttransfusionen erheblich zu mindern, Knochenmarktransplantationen einfacher und risikoärmer zu gestalten, als auch die Immunabwehr gegenüber Krankheitserregern (einschließlich AIDS) oder auch Krebs zu unterstützen.

Die erfindungsgemäßen Spleninderivate der Formel I können durch Vermischen mit pharmakologisch unbenklichen Trägermaterialien und / oder Verdünnungsmitteln in pharmazeutische Verabreichungsformen überführt werden.

Zum Nachweis der immunologischen Wirkungen wurden folgende Modelle verwendet:
- "Kolonien - Bildung" ("Kolonien" - Bildungstest ) durch menschliche Knochenmarkzellen nach Kultivierung der Zellen mit dem menschlichen Granulocyten - Makrophagen - "Kolonien" -stimulierenden Faktor (hGM-CSF) und Spleninderivaten.
- die Antikörperbildung gegen Schafserythrozyten ( Plaquebildende Milzzellen ) durch Ab/Bln.-Mäuse nach Röntgenbestrahlung (Tabelle 2).

Die erfindungsgemäßen Spleninderivate weisen wertvolle pharmakologische Eigenschaften, insbesondere solche zur Normalisierung des Immunsystems, auf. Daher eignen sie sich zur Anwendung bei folgenden medizinischen Indikationen:

- Behandlung viraler Infektionen

Die Spleninderivate, insbesondere die acylierten Spleninderivate, haben sich als geeignete Wirkstoffe gegen Influenzaviren oder zur Pankreatitis-Behandlung nach Infektion mit Coxsackieviren erwiesen. Hinsichtlich des Ausmaßes der Stimulierung der antiviralen Immunabwehr wurde festgestellt, daß bei Gabe von Virusmengen, die zu einer Infektion mittlerer Schwere führen, die Erkrankung vollständig verhindert werden kann und daß die zur Auslösung einer Erkrankung gleichen Schweregrades notwendige Virusdosis unter der Behandlung um ein Vielfaches höher ist als bei Individuen ohne Therapie.

- Behandlung nach Chemotherapie

Die pharmazeutischen Mittel auf der Basis der erfindungsgemäßen Spleninderivate sind geeignet zur überwindung immunsuppressiver Zustände nach chemotherapeutischen Maßnahmen, z.B. bei der Krebsbehandlung oder infolge überdosierter Ciclosporin-Therapie

Die Normalisierung der humoralen Immunantwort nach Cyclophosphamid- oder Dexamethason-Behandlung von Mäusen läßt sich durch die Gabe der erfindungsgemäßen Spleninderivate erreichen. Da diese jedoch keinen Einfluß auf die Ausbildung der normalen Antigen-spezifischen humoralen Immunantwort haben, sind keine nachteiligen immunmodulatorischen Nebenwirkungen zu erwarten.

Die Spleninderivate bewirken die beschleunigte Rekonstitution des Immunsystems von Patienten mit sekundärer Immundefizienz als Folge immunsuppressiver Therapie.

- Stimulierung des Wachstums und der Reifung von Knochenmarkzellen.

Die Anwendung der erfindungsgemäß hergestellten Verbindungen führt in vitro zu einer Erhöhung der Bildungsrate von Stammzellkolonien aus Maus-Knochenmarkzellen, die mit Leukozyten von Normalpersonen oder Patienten mit Systemischem Lupus Erythematodes in Agar ko-kultiviert wurden. Die vermehrte Bildung von "Kolonien" als Folge der Gegenwart der Spleninderivate ist ein Hinweis dafür, daß durch diese direkt die Proliferation unreifer Stammzellen des Knochenmarks angeregt wird und / oder Leukozyten des Menschen unter dem Einfluß der Spleninderivate vermehrt koloniebildende Faktoren sezermieren.
Bei subletal bestrahlten und mit syngenen Knochenmarkzellen transplantierten Mäusen ist eine beschleunigte Rekonstitution der humoralen Immunantwort zu beobachten.
Die Wirkstoffe sind bei immunsuppressiver bzw. cytostatischer Therapie, nach Bestrahlung und nach Knochenmarktransplantationen anwendbar. Sie wirken dadurch, daß sie spezifische Bindungsorte (Rezeptoren) auf Knochenmarkzellen des Menschen (insbesondere auf Stammzellen) besitzen, die durch Radioligand-Bindungsstudien nachgewiesen werden können. Derartige Bindungsstellen finden sich auch auf Thymozyten und können durch $^3$H-markierte Spleninderivate oder durch $^{125}$I-markierte, insbesondere diacylierte Spleninderivate nachgewiesen werden.

- Therapie von HIV-Infektionen

Die Gabe der erfindungsgemäßen Spleninderivate, insbesondere der diacylierten Verbindungen, bewirkt bei HIV-infizierten Patienten eine Anhebung der Gesamtlymphozytenzahl in den Kontrollbereich.
Eine Kombination mit dem in der AIDS-Therapie eingesetzten Zytostatikum und Revertasehemmer Azido-Thymidin sowie wirksameren Substanzen dieses Typs (Fluor-Thymidin) führt zu einer beschleunigten Normalisierung der durch diese Substanzen bewirkten nachteiligen Veränderungen im Immunsystem.

-Verhinderung immunsuppressiver Effekte chronischer Intoxikationen

Die Wirkung der erfindungsgemäßen Verbindungen besteht darin, daß die durch Alkohol und andere chronisch wirkende Noxen induzierte Suppression der Antigen-spezifischen humoralen Immunreaktion sowie die Verminderung der Zahl bzw. Aktivität phagozytierender Zellen aufgehoben und die partielle Atropie des Thymus und der Milz verhindert wird.

- Therapie von Autoimmunreaktionen

Die erfindungsgemäß hergestellten Substanzen eignen sich zur Therapie verschiedener Krankheiten mit einer Autoimmunkomponente, wie der Rheumatoidarthritis, SLE und atopischen Reaktionen. Sie bewirken jedoch nicht die nachteilige Reduktion des Antigen-spezifischen Antikörpertiters nach erfolgter Immunisierung.

Ausführungsbeispiele

Beispiel 1

1. Herstellung von (N$^\alpha$-Acetyl)-arginyl-(N$^\epsilon$-acetyl)-lysylalanylvalyltyrosin (DAc - h - SP-5)

1.1 Synthese von Boc-Val-Tyr-OBzl

1,2g (5,5 mMol) Boc-Val-OH werden in DMF unter Zusatz von 0,65ml (5,5mMol) NMM mit 0,68ml (5,25mMol) CAIBE bei -15°C zum Mischanhydrid aktiviert. Dazu gibt man die gekühlte Lösung von 2,22g (5 mMol) Tyr-OBzl*Tos in DMF unter Zusatz von 0,55ml (5 mMol) NMM. Nach beendeter Reaktion wird Lösungsmittel im Vak. entfernt. Den Rückstand nimmt man in EE auf und schüttelt die organische Phase sauer und alkalisch aus, trocknet über $Na_2SO_4$, engt im Vak. ein und fällt mit Ether. Der Feststoff wird abgesaugt, mit Ether gewaschen und im Vak. getrocknet.
Ausbeute: 2,2g / 93,5% d. Th.
MG: 470,58
Summenformel: $C_{26}H_{34}N_2O_6$

### 1.2 Freisetzung zum H-Val-Tyr-OBzl*HCl

2g (4,25mMol) Boc-Val-Tyr-OBzl werden 30 min mit 10,6ml 4n HCl/EE behandelt. Die Überschüssige HCl/EE wird im Vak abdestilliert und das Produkt aus EE gefällt, abfiltriert, mit EE gewaschen und im Vak. über KOH getrocknet.
Ausbeute: 1.37g / 79,3% d. Th.
MG: 406,91
Summenformel: $C_{21}H_{27}N_2O_4Cl$

### 1.3 Synthese von Boc-Ala-Val-Tyr-OBzl

1,02g (5,41mMol) Boc-Ala-OH werden in DMF unter Zusatz von 0,59ml (5,4mMol) NMM bei -15°C mit 0,67ml (5,16mMol) CAIBE aktiviert und anschließend mit der vorgekühlten Lösung von 2,0g (4,92m Mol) H-Val-Tyr-OBzl*HCl und 0,54ml (4,92mMol) NMM zur Reaktion gebracht. Aufarbeitung erfolgt analog 1.1 .
Ausbeute: 2,5g / 92,4% d.Th.
MG: 541,66
Summenformel: $C_{29}H_{39}N_3O_7$

### 1.4 Freisetzung zum H-Ala-Val-Tyr-OBzl*HCl

2,46g (4,54mMol) Boc-Ala-Val-Tyr-OBzl werden mit 12,5 ml (50mMol) 4n HCl/EE behandelt. Die Überschüssige HCl/EE wird im Vak. abdestilliert und Produkt mit Ether gefällt, abfiltriert, mit Ether gewaschen und im Vak. über KOH getrocknet.
Ausbeute: 1.95g / 89,9% d.Th.
MG: 478,0
Summenformel: $C_{24}H_{32}N_3O_5Cl$

### 1.5 Synthese von Boc-Lys(Z)-Ala-Val-Tyr-OBzl

1,9g (5mMol) Boc-Lys(Z)-OH werden in DMF unter Zusatz von 0,55ml (5mMol) NMM bei -15°C mit 0,62ml (4,77mMol) CAIBE aktiviert und anschließend mit der vorgekühlten Lösung von 2,17g (4,54mMol) H-Ala-Val-Tyr-OBzl*HCl und 0,5ml (4,54mMol) NMM zur Reaktion gebracht. Nach beendeter Reaktion wird die Reaktionslösung in 5%ige $NaHCO_3$ eingerührt. Das Produkt wird abfiltriert und neutral gewaschen, anschließend erneut in DMF gelöst und in 1n HCl eingerührt, filtriert und neutral gewaschen und getrocknet.
Ausbeute: 3,47g / 95% d.Th.
MG: 803,92
Summenformel: $C_{43}H_{57}N_5O_{10}$

### 1.6 Freisetzung zum H-Lys(Z)-Ala-Val-Tyr-OBzl*TFA

3,65g (4,54mMol) Boc-Lys(Z)-Ala-Val-Tyr-OBzl werden 1h mit 17,3ml (227mMol) TFA in 15ml Dichlorethan behandelt. Nach Abdestillieren der Überschüssigen TFA/Dichlorethan wird der Rückstand in wenig Dichlorethan aufgenommen und in Ether eingerührt, Produkt abfiltriert, mit Ether gewaschen und im Vak. über KOH getrocknet.
Ausbeute: 3,23g / 87% d. Th.
MG: 817,88
Summenformel: $C_{40}H_{50}N_5O_{10}F_3$

1.7 Synthese von Boc-Arg(NO$_2$)-Lys(Z)-Ala-Val-Tyr-OBzl

1,39g (4,35mMol) Boc-Arg(NO$_2$)-OH werden in DMF unter Zusatz von 0,48ml (4,35mMol) NMM bei -15°C mit 0,54ml (4,15mMol) CAIBE aktiviert und mit der vorgekühlten Lösung von 3,23g (3,95mMol) H-Lys(Z)-Ala-Val-Tyr-OBzl*TFA und 0,44ml (3,95mMol) NMM zur Reaktion gebracht. Die Aufarbeitung erfolgt analog 1.5 .
Ausbeute: 3,48g / 87,6% d.Th.
MG: 1005,18
Summenformel: C$_{49}$H$_{69}$N$_{10}$O$_{13}$

1.8 Freisetzung zum H-Arg(NO$_2$)-Lys(Z)-Ala-Val-Tyr-OBzl*TFA

3,48g (3,46mMol) Boc-Arg(NO$_2$)-Lys(Z)-Ala-Val-Tyr-OBzl werden 1h mit 13,15ml (173mMol) TFA in 12ml Dichlorethan behandelt. Die Aufarbeitung erfolgt analog 1.6 .
Ausbeute: 3,4g / 96,4% d. Th.
MG: 1o19,07
Summenformel: C$_{46}$H$_{61}$N$_{10}$O$_{13}$F$_3$

1.9 Hydrierung zum H-Arg-Lys-Ala-Val-Tyr-OH * 3AcOH

3,0g (2,94mMol) H-Arg(NO$_2$)-Lys(Z)-Ala-Val-Tyr-OBzl*TFA werden in 90%iger AcOH gelöst, 0,3g Pd/A-Kohle (10%ig) zugesetzt und bei RT und Normaldruck H$_2$-Gas eingeleitet. Nach vollständiger Schutzgruppenabspaltung wird Katalysator abfiltriert, die Lösung eingeengt und in Ether eingerührt. Das Produkt wird abfiltriert, mit Ether gewaschen und getrocknet.
Ausbeute: 2,28g 95% d. Th.
MG: 815,78
Summenformel C$_{35}$H$_{61}$N$_9$O$_{13}$

1.10 Acetylierung zum Ac-Arg-Lys(Ac)-Ala-Val-Tyr-OH*AcOH

0,816g (1mMol) Arg-Lys-Ala-Val-Tyr * 3AcOH, gelöst in 10ml Wasser, werden mit einer Lösung von 0,66g (3mMol) AcONB (N-Acetoxy-norborn-5-en-2,3-dicarboximid) in 10ml DMF zur Reaktion gebracht. Nach beendeter Reaktion wird das Lösungsmittelgemisch im Vak. eingeengt und der Rückstand in Ether eingetropft. Das Produkt wird abfiltriert, mit Ether gewaschen und im Vak. getrocknet.
Ausbeute: 0,7g / 90% d. Th.
MG: 779,91
Summenformel: C$_{35}$H$_{57}$N$_9$O$_{11}$

Beispiel 2

1. Herstellung von (N$^\alpha$-Acetyl-)arginyllysylalanylvalyltyrosin ($\alpha$-MAc-SP-5*2AcOH)

1.1 Synthese von Ac-Arg(NO$_2$)-Lys(Z)-Ala-Val-Tyr-OBzl

1,09g (1mMol) H-Arg(NO$_2$)-Lys(Z)-Ala-Val-Tyr-OBzl*TFA werden in 5ml DMF gelöst und nach Zugabe von 180 l NMM (oder einem anderen tertiären Amin) mit 0,27g (1,2mMol) AcONB umgesetzt. Nach 30min der Niederschlag abgesaugt und mit Wasser gewaschen. Das Produkt wird in ca. 10ml DMF gelöst und mit 5%iger NaHCO$_3$-Lösung gefällt, filtriert, mit Wasser gewaschen und getrocknet.
Ausbeute: 0,92g / 96,6% d. Th.
MG: 947,01
Summenformel: C$_{46}$H$_{69}$N$_{10}$O$_{12}$

1.2 Hydrierung zum Ac-Arg-Lys-Ala-Val-Tyr-OH*2AcOH

0,9g (0,95mMol) Ac-Arg($NO_2$)-Lys(Z)-Ala-Val-Tyr-OBzl werden analog Ausführungsbeispiel 3 Pkt.1.9 hydriert.

Ausbeute: 0,7g / 92% d.Th.

MG: 797,93

Summenformel $C_{35}H_{59}N_9O_{12}$

Beispiel 3

1. Herstellung von H-Arg-Lys(Ac)-Ala-Val-Tyr-OH*2AcOH

1.1 Hydrierung zum Boc-Arg-Lys-Ala-Val-Tyr-OH*2AcOH analog zum Beispiel 3 Pkt. 1.9 werden 5,02g (5mMol) Boc-Arg($NO_2$)-Lys(Z)-Ala-Val-Tyr-OBzl hydriert.

Ausbeute: 3,98g / 93%

MG: 856

Summenformel: $C_{38}H_{65}N_9O_{13}$

1.2 Acetylierung zum Boc-Arg-Lys(Ac)-Ala-Val-Tyr-OH*AcOH analog zum Beispiel 3 Pkt. 1.10 werden 3,42g (4mMol) Boc-Arg-Lys-Ala-Val-Tyr-OH*2AcOH acetyliert.

Ausbeute: 2,98g/ 89% d. Th.

MG: 837,98

Summenformel: $C_{38}H_{63}N_9O_{12}$

1.3 Deblockierung zum H-Arg-Lys(Ac)-Ala-Val-Tyr-OH*2AcOH analog zum Beispiel 3 Pkt. 1.8 werden 2,51g (3mMol) Boc-Arg-Lys(Ac)-Ala-Val-Tyr-OH*AcOH deblockiert.Nach vollständiger Deblockierung wird Reaktionsgemisch im Vak. eingeengt, Rückstand in Eisessig aufgenommen und in Ether eingetropft, filtriert, gewaschen mit Ether und im Vak. getrocknet.

Ausbeute: 2,03g / 85% d. Th.

MG: 797,93

Summenformel: $C_{35}H_{59}N_9O_{12}$

Beispiel 4

1. Herstellung von Ac-Arg-Lys(Ac)-Ala-OH*AcOH

1.1 Synthese von Boc-Lys(Z)-Ala-OBzl

MA-Synthese analog Ausführungsbeispiel 3 Pkt 1.1 von Boc-Lys(Z)OH mit H-Ala-OBzl*HCl

Ausbeute: ca.75% d. Th.

MG: 541,62

Summenformel: $C_{29}H_{39}N_3O_7$

1.2 Freisetzung zum H-Lys(Z)-Ala-OBzl*HCl

Deblockierung analog Beispiel 3 Pkt.1.2 von Boc-Lys(Z)-Ala-OBzl

Ausbeute: Öl ca. 90%

MG: 478,01

Summenformel: $C_{24}H_{32}N_3O_5CL$

1.3 Synthese von Boc-Arg($NO_2$)-Lys(Z)-Ala-OBzl

MA-Synthese analog Beispiel 3 Pkt. 1.1 von Boc-Arg($NO_2$)-OH mit H-Lys(Z)-Ala-OBzl*HCl

Ausbeute: 80% d. Th.

MG: 742,85

Summenformel $C_{35}H_{50}N_8O_{10}$

1.4 Freisetzung zum H-Arg($NO_2$)-Lys(Z)-Ala-OBzl*HCl

Deblockierung analog Beispiel 3 Pkt. 1.4 von Boc-Arg($NO_2$)-Lys(Z)-Ala-OBzl

Ausbeute: 80% d. Th.

MG: 679,23

Summenformel: $C_{30}H_{43}N_8O_8Cl$

8

1.5 Hydrierung zum H-Arg-Lys-Ala-OH*3AcOH
analog Beispiel 3 Pkt. 1.9 wird H-Arg($NO_2$)-Lys(Z)-Ala-OBzl*HCl hydriert
Ausbeute: 91% d. Th.
MG: 553,62
Summenformel: $C_{21}H_{43}N_7O_{10}$
1.6 Acetylierung zum Ac-Arg-Lys(Ac)-Ala-OH*AcOH
analog Beispiel 3 Pkt. 1.10 wird H-Arg-Lys-Ala-OH*3AcOH acetyliert.
Ausbeute: 88% d. Th.
MG: 517,59
Summenformel: $C_{21}H_{39}N_7O_5$

Beispiel 5

1. Herstellung von Ac-Arg-Lys-Ala-OH*2AcOH

1.1 Acetylierung zum Ac-Arg($NO_2$)-Lys(Z)-Ala-OBzl
analog Beispiel 4 Pkt. 1.1 wird H-Arg($NO_2$)-Lys(Z)-Ala-OBzl*HCl (Bs.p.6.1.4) acetyliert.
Ausbeute: 87% d.Th.
MG: 684,77
Summenformel: $C_{32}H_{44}N_8O_9$
1.2 Hydrierung zum Ac-Arg-Lys-Ala-OH*2AcOH
analog Beispiel 3 Pkt. 1.9 wird Ac-Arg($NO_2$)-Lys(Z)0Ala-OBzl hydriert
Ausbeute: 92% d. Th.
MG: 535,59
Summenformel: $C_{21}H_{41}N_7O_9$

Beispiel 6

1. Herstellung von H-Arg-Lys(Ac)-Ala-OH*2AcOH

1.1 Hydrierung zum Boc-Arg-Lys-Ala-OH*2AcOH
analog Beispiel 3 Pkt. 1.9 wird Boc-Arg($NO_2$)-Lys(Z)-Ala-OBzl*2AcOH (Bsp. 6.1.3) hydriert.
Ausbeute: 90% d. Th.
MG: 593,69
Summenformel: $C_{24}H_{47}N_7O_{10}$
1.2 Acetylierung zum Boc-Arg-Lys(Ac)-Ala-OH*AcOH
analog Beispiel 3 Pkt. 1.10 wird Boc-Arg-Lys-Ala-OH*2AcOH acetyliert
Ausbeute: 87% d.Th.
MG: 575,68
Summenformel: $C_{24}H_{45}N_7O_9$
1.3 Deblockierung zum Arg-Lys(Ac)-Ala-OH*2AcOH
Nach Deblockierung von Boc-Arg-Lys(AC)-Ala-OH*2AcOH mit TFA/Dichlorethan (1:1) engt man Reaktionsmischung im Vak. ein, löst Rückstand in Eisessig und tropft in Ether ein.
Ausbeute: 86% d. Th.
MG: 535,59
Summenformel: $C_{21}H_{41}N_7O_9$

Beispiel 7

Herstellung der Peptid-Hydrochloride

Durch Auflösen der in den Beispielen 3.1.9, 3.1.10, 4.1.2, 5.1.3, 6.1.5, 6.1.6, 7.1.2 und 8.1.3 beschriebenen Peptide in 1,1 Äquivalenten 0,1n HCl und nachfolgende Lyophilisation werden die entsprechenden Peptid-Hydrochloride erhalten.

Beispiel 8

Herstellung der freien Peptide

Durch Auflösen der in den Beispielen 3.1.9, 3.1.10, 4.1.2, 5.1.3, 6.1.5, 6.1.6, 7.1.2 und 8.1.3 oder in Beispiel 9 beschriebenen Peptide in Wasser und Titration mit 0,1n NaOH werden nach säulenchromatographischer Entsalzung und Lyophilisation die entsprechenden freien Peptide erhalten.

Beispiel 9

Stimulierung des Wachstums und der Reifung von Knochenmarkstammzellen (in vitro)

Tabelle 1

Anzahl der Kolonien pro 10⁵ menschlicher Knochenmarkzellen (KMZ) nach Kultivierung [5] der Zellen mit dem menschlichen Granulocyten-Makrophagen-Kolonien-stimulierenden Faktor (hGM-CSF)[6] und Splenopentin (SP5), Splenopentin-Analoga oder Thymopentin (TP5)

| Nr. | Peptid [1] | | Anzahl der Kolonien pro 10⁵ KMZ [2] | | p [3] |
|-----|-----------|---|------|------|-------|
| 1 | nur hGM-CSF | | 46,7 | 5,6 | |
| 2 | hGM-CSF | | | | |
| | bSP5: | 1μg × ml⁻¹ | 46,5 | 9,4 | n.s.[4] |
| | | 10μg × ml⁻¹ | 69,0 | 11,9 | 0,01 |
| 3 | hGM-CSF | | | | |
| | DAc-bSP5: | 1μg × ml⁻¹ | 92,0 | 21,6 | 0,001 |
| | | 10μg × ml⁻¹ | 56,0 | 8,7 | n.s. |
| 4 | hGM-CSF | | | | |
| | hSP5: | 1μg × ml⁻¹ | 70,5 | 11,4 | 0,01 |
| | | 10μg × ml⁻¹ | 77,5 | 7,5 | 0,01 |
| 5 | hGM-CSF | | | | |
| | DAc-hSP5: | 1μg × ml⁻¹ | 116,2 | 23,7 | 0,01 |
| | | 10μg × ml⁻¹ | 66,7 | 7,9 | n.s. |
| 6 | hGM-CSF | | | | |
| | TP5: | 1μg × ml⁻¹ | 51,2 | 7,3 | n.s. |
| | | 10μg × ml⁻¹ | 48,3 | 3,9 | n.s. |
| | | 100μg × ml⁻¹ | 48,2 | 4,4 | n.s. |

1) bSP5 (bovines Splenopentin): H-Arg-Lys-Glu-Val-Tyr-OH

DAc-hSP5: $(N^\alpha$-Acetyl-Arginyl)-$(N^\iota$-Acetyl-Lysyl)-Glutamyl-
Valyl-Tyrosin-OH

$(N^\alpha$-Acetyl-Arginyl)-$(N^\varepsilon$-Acetyl-Lysyl)-Glutamyl-
Valyl-Tyrosin-HCl

hSP5 (humanes Splenopentin): H-Arg-Lys-Ala-Val-Tyr-OH

DAc-hSP5: $(N^\alpha$-Acetyl-Arginyl)-$(N^\varepsilon$-Acetyl-Lysyl)-Alanyl-
Valyl-Tyrosin-OH

TP5: (Thymopentin): H-Arg-Lys-Asp-Val-Tyr-OH

2) Die Werte repräsentieren den Mittelwert $\pm$ Streuung von Dreifachbestimmungen von Knochenmarkzellen erhalten von drei differenten Spendern

3) Statistische Differenzen: (Mann-Whitney-Test): Vergleich der Werte Nr.2 bis Nr.6 mit Nr.1

4) n.s.= nicht signifikanter Unterschied.

5) Methodik:Spooncer et.al., Differentation,1986,(31),111

6) Produkt der Behring-Werke Marburg

Die Anzahl der gebildeten "Kolonien" ist dabei ein Maß für die mitotische bzw. differenzierende Wirkung der Faktoren auf die Knochenmarkzellen.

Da Thymopentin in diesem Testsystem (Tab.1, Nr.6) keine Wirkung zeigte, ist anzunehmen, daß die immunstimulierende Wirkung von Timunox nicht über eine Wirkung auf Stammzellen des Knochenmarks zustande kommt.

Beispiel 10

Beeinflussung der Funktion von Zellen des Knochenmarks (in vivo)

Es wurden drei Monate alte AB/Bln.-Mäuse einmalig mit 800 cGy röntgenbestrahlt. Danach wurden die Tiere in drei Gruppen aufgeteilt und folgendermaßen weiterbehandelt:

Gruppe 1: Nach der Bestrahlung wurde den Tieren Knochenmarkzellen von Tieren des gleichen Inzuchtstammes transplantiert (Injektion von je 5 mal $10^6$ Knochenmarkzellen pro Tier). Anschließend wurden die Tiere dreimal wöchentlich mit DAc-hSP5 in einer Dosis von 10 µg pro Tier behandelt (intraperitoneale Applikation). In differenten Zeitabständen nach der Bestrahlung und während der DAc-hSP5-Behandlung wurde mittels des JERNE-Plaque-Testes die Fähigkeit der Tiere untersucht, Antikörper zu bilden.

Gruppe 2: Gleiche Behandlung der Tiere wie in Gruppe 1, jedoch keine Therapie der Tiere mit DAc-hSP5.

Gruppe 3: Alleinige Röntgenbestrahlung der Tiere; keine Transplantation syngener Knochenmarkzellen, keine Therapie der Tiere mit DAc-hSP5.

Fünf Tage vor der Bestimmung der IgM Plaque-bildenden Milzzellen (Tage, angegeben in Tabelle 2) wurden die Tiere mit $5 \times 10^8$ Schafserythrozyten immunisiert (intaperitoneale Applikation). Methodik: Eckert et.al., Biomed. Biochem. Acta, 1985, (44), 1239.

Die Ergebnisse zeigen (Tabelle 2), daß unter in vivo Bedingungen Zellen des Knochenmarks durch DAc-hSP5 beschleunigt zu einer normalen Antikörperbildung befähigt werden. Dies impliziert, daß durch DAc-hSP5 Zellen des Knochenmarks auch in vivo beschleunigt "reifen", d.h. sich schneller in funktionell aktive Zellen differenzieren.

Die intraperitoneale Applikation von 10 ug pro Tier dreimal wöchentlich hatte einen optimalen Effekt, 1 $\mu$g, 2 $\mu$g und 4 $\mu$g waren unter identischen Bedingungen nicht wirksam, 8 $\mu$g wirkten suboptimal. Nach Applikation von 20 $\mu$g und 50 $\mu$g konnte ein Effekt wie nach der von 10 ug gemessen werden. Grundsätzlich gleichartige Ergebnisse wurden bei der Verwendung anderer Spleninderivate der Formel I erzielt.

Tabelle 2

Antikörperbildung gegen Schafserythrozythen durch AB/Bln.-Mäuse nach einmaliger Röntgenbestrahlung der Tiere mit 800 cGy, nachfolgender Transplantation syngener Knochenmarkzellen (5 x 10⁶ Knochenmarkzellen) und anschließender Behandlung der Tiere mit DAc-hSP5 (Nr.3). Gabe von 10 ug DAc-hSP5 untägig dreimal wöchentlich. Jeder Wert (Mittelwert Streuung) repräsentiert den Mittelwert von mindestens fünf Tieren.

| Nr. | Behandlung | Anzahl Plaque-bildender Milzzellen pro Milz | | | |
|-----|-----------|--------|--------|--------|--------|
| | | Tag 14 | Tag 20 | Tag 34 | Tag 48 |
| 1 | Röntgenbestrahlung | 1500 ± 500 | 1400 ± 400 | 11000 ± 2400 | 17000 ± 2600 |
| 2 | Röntgenbestrahlung und Knochenmark - transplantation | 1600 ± 300 | 9500 ± 1100 | 15800 ± 1600 | 28000 ± 4300 |
| 3 | Röntgenbehandlung und Knochenmark - transplantation und DAc-hSP5 (10 ug) | 2900 ± 300 | 16000 ± 1000 | 39000 ± 8500 | 44000 ± 7000 |
| 4 | ohne Röntgenbestrahlung ohne jegliche Behand - lung (Kontrolle) | 43100 ± 4600 | 41000 ± 3000 | 43000 ± 7000 | 42500 ± 8300 |

U-Test (Mann-Whitney): Vergleich zwischen Nr.1 und 2 : Tag 20: $p < 0,001$

Vergleich zwischen Nr.2 und 3 : Tag 20: $p < 0,001$

Vergleich zwischen Nr.3 und 4 : Tag 34: kein signifikanter Unterschied

Abkürzungsverzeichnis

AcOH - Essigsäure
AcONB - N-Acetoxy-norborn-5-en-2,3-dicarboximid
CAIBE - Chlorameisensäureisobutylester
DMF - Dimethylformamid
EE - Essigsäureethylester
NMM - N-Methylmorpholin
TFA - Trifluoressigsäure


**Ansprüche**

1. Verfahren zur Herstellung von humanen Spleninderivaten der Formel I

Arg - X - Ala - R$^1$    I,

worin

Arg ggf. substituiertes Arginin,

X Sar, Pro, Gly, Ala, Leu, Ile, Thr, Ser, Lys, Val, Phe oder Cys, die ggf. substituiert sind,

R$^1$ -O R$^3$, -N (R$^3$ R$^4$), -X-Z-O R$^3$ oder -X-Z-N (R$^3$ R$^4$),

worin

R$^3$ und R$^4$ gleich oder verschieden sind und H, $C_1$-$C_{18}$-Alkyl, $C_2$-$C_7$ - Alkenyl, $C_2$-$C_7$ - Alkinyl, $C_7$-$C_{20}$ - Alkaryl, $C_7$-$C_{20}$ - Aralkyl oder Polyhydroxyalkyl

und

Z Tyr, Phe, decarboxy-Tyr, decarboxy-Phe , die ggf. durch OH, NO$_2$, Halogen und OCH$_3$ substituiert sind oder Trp

bedeuten,

die Aminosäuren jeweils die L - oder D - Konfiguration aufweisen können,

sowie deren durch Verknüpfung der α- bzw. ε-Aminogruppe mit der α- Carboxylgruppe des Alanins bzw. der Aminosäure Z direkt oder mit Dicarbonsäuren

und/oder durch Verknüpfung der - Carboxylgruppe des Alanins bzw. der Aminosäure Z über Polyoxyethylen-, Polyoxyethylendiamino-oder Polymethylendiamino-Ketten

und/oder durch Verknüpfung über S - S - Brückenbindung am Cystein erhaltene Dimere,

sowie deren durch Verknüpfung der Carboxylgruppe der Aminosäure Z mit der Aminogruppe des Arginins erhaltenen cyclischen Derivate,

sowie deren Salze, Komplexe, Amide, Ester und entsprechenden, ggf. partiell, geschützten Derivate, gekennzeichnet dadurch, daß man die entsprechenden, ggf. geschützten und/oder acylierten Aminosäuren bzw. deren Salze, Komplexe, Amide oder Ester stufenweise oder durch Fragmentkondensation nach an sich bekannten Methoden der Peptidsynthese zu den ggf. geschützten Spleninderivaten der Formel I bzw. deren Salzen, Komplexen, Amiden und Estern umsetzt,

ggf. in an sich bekannter Weise die Schutzgruppen selektiv abspaltet

ggf. in an sich bekannter Weise acyliert, cyclisiert bzw. dimerisiert,

ggf. die, ggf. geschützten Spleninderivate der Formel I in ihre Salze, Komplexe, Amide oder Ester überführt und/oder aus den Salzen,Komplexen, Amiden und Estern das freie Peptid der Formel I freisetzt.

2. Verfahren nach Anspruch 1, gekennzeichnet dadurch, daß die Acylierung der ggf. geschützten Spleninderivate der Formel I und der entsprechenden, ggf. geschützten Aminosäuren bzw. der entsprechenden, ggf. geschützten Di-, Tri- und Tetrapeptidfragmente mit N- Hydroxy - norborn-5-en-2,3- dicarboximidestern von Carbonsäuren, vorzugsweise N- Acetoxy-norborn-5-en-2,3- dicarboximid durchgeführt wird.

3.Verfahren nach den Ansprüchen 1 und 2, gekennzeichnet dadurch, daß die Acylierung der ggf. geschützten Spleninderivate der Formel I und der entsprechenden, ggf. geschützten Aminosäuren bzw. der entsprechenden, ggf. geschützten Di- , Tri- und Tetrapeptidfragmente mit N -Acetoxybenzotriazol durchgeführt wird.

4. Verfahren nach den Ansprüchen 1 bis 3, gekennzeichnet dadurch, daß Spleninderivate der Formel I , worin die Aminogruppe des Arginins und/oder der Aminosäure X, insbesondere des Lysins, substituiert ist durch eine der folgenden Gruppierungen:

$C_1$-$C_7$ - Alkyl, $C_6$-$C_{12}$ - Aryl, $C_7$-$C_{20}$ - Alkaryl, $C_7$-$C_{20}$ - Aralkyl, $C_2$-$C_7$ - Alkenyl, $C_2$-$C_7$ - Alkinyl, $C_3$-$C_7$ - Cycloalkyl, $C_3$-$C_7$ -Cycloalkenyl, $C_1$-$C_{18}$ - Alkanoyl, $C_4$-$C_{12}$ -Cycloalkanoyl, $C_7$-$C_{11}$ -Aroyl, $C_6$-$C_{12}$ - Polydroxyalkanoyl, $C_7$-$C_{24}$ - Aralkanoyl,

- CO(CH$_2$)$_m$ -CO-R$^5$, - CO(CHOH)$_n$-CO-R$^5$, mit m = 1-8 und n = 1-12,

14

wobei $R^5$ OH bzw.deren entsprechende Salze, Ester, und Amide, $N^\alpha$ - Arg - X - Ala - $R^1$ oder $N^\epsilon$ - Lys - (Arg) - Ala - $R^1$ bedeutet, hergestellt werden.

5.Verfahren nach den Ansprüchen 1 bis 4, gekennzeichnet dadurch, daß solche Dimeren der Spleninderivate der Formel I hergestellt werden, die durch Verknüpfung der -Carboxylgruppe des Alanins bzw. der Aminosäure Z über eine der folgenden Gruppierungen erhalten werden:

-O-$(CH_2)_p$-O-,

-NH-$(CH_2)_p$-NH-,

-O-$(CH_2$-$CH_2$-O-$)_q$-,

-NH-$(CH_2$-$CH_2$-O-$)_q$-$CH_2$-$CH_2$-NH- mit p = 1-18 und q = 1-10 000 .

6. Verfahren nach den Ansprüchen 1 bis 5, gekennzeichnet dadurch, daß Spleninderivate der Formel I , worin X Lys und $R^1$ Val-Tyr- $OR^3$ oder Val-Tyr-N $(R^3R^4)$ bedeuten und Arg und/oder Lys und/oder Val-Tyr substituiert sind, hergestellt werden.

7. Verfahren nach den Ansprüchen 1 bis 6, gekennzeichnet dadurch, daß Spleninderivate der Formel I hergestellt werden, die aus der folgenden Gruppe ausgewählt sind:

$(N^\alpha$ -Acyl-Arg) - X - Ala - $R^1$

Arg -$(N^\epsilon$-Acyl- Lys) - Ala-$R^1$

$(N^\alpha$-Acyl-Arg) - $(N^\epsilon$-Acyl- Lys) - Ala - $R^1$ ,

wobei Acyl bevorzugt Acetyl, X bevorzugt Lys und $R^1$ bevorzugt Val-Tyr-$OR^3$ oder Val-Tyr-N $(R^3 R^4)$ bedeuten, Valin und Tyrosin ggf. substituiert sind.

8.Verfahren zur Herstellung von $(N^\alpha$-Acyl-Arg)-$(N^\epsilon$-Acyl-Lys)-Ala-$R^1$ nach den Ansprüchen 1 bis 7, gekennzeichnet dadurch, daß Arg-Lys-Ala-$R^1$ acyliert wird.

9. Verfahren zur Herstellung von Arg -$(N^\epsilon$-Acyl- Lys) - Ala - $R^1$ und $(N^\alpha$- Acyl-Arg) - Lys - Ala - $R^1$ nach den Ansprüchen 1 bis 7, gekennzeichnet dadurch, daß partiell geschütztes Arg-Lys-Ala-$R^1$ acyliert und anschließend die $N^\alpha$ - bzw. $N^\epsilon$ - Schutzgruppe abgespalten wird.

10. Humane Spleninderivate der Formel I

Arg - X - Ala - $R^1$      I ,

worin

Arg ggf.substituiertes Arginin,

X Sar, Pro, Gly, Ala, Leu, Ile, Thr, Ser, Lys, Val, Phe oder Cys, die ggf. substituiert sind,

$R^1$ -O $R^3$, -N $(R^3 R^4)$, -X-Z-O $R^3$ oder -X-Z-N $(R^3 R^4)$,

worin

$R^3$ und $R^4$ gleich oder verschieden sind und H, $C_1$-$C_{18}$ - Alkyl, $C_2$-$C_7$ - Alkenyl , $C_2$-$C_7$ - Alkinyl, $C_7$-$C_{20}$ - Alkaryl, $C_7$-$C_{20}$ - Aralkyl oder Polyhydroxyalkyl

und

Z Tyr, Phe, decarboxy-Tyr, decarboxy-Phe , die ggf. durch OH, $NO_2$, Halogen und $OCH_3$ substituiert sind, oder Trp

bedeuten,

die Aminosäuren jeweils die L - oder D - Konfiguration aufweisen können,

sowie deren durch Verknüpfung der α- bzw. ε- Aminogruppe mit der α- Carboxylgruppe des Alanins bzw. der Aminosäure Z direkt oder mit Dicarbonsäuren

und/oder durch Verknüpfung der α- Carboxylgruppe des Alanins bzw. der Aminosäure Z über Polyoxyethylen-, Polyoxyethylendiamino-oder Polymethylendiamino- Ketten

und/oder durch Verknüpfung über S - S - Brückenbindung am Cystein erhaltene Dimere,

sowie deren durch Verknüpfung der Carboxylgruppe der Aminosäure Z mit der Aminogruppe des Arginins erhaltenen cyclischen Derivate,

sowie deren Salze, Komplexe, Amide, Ester und entsprechenden , ggf. partiell, geschützten Derivate.

11. Spleninderivate nach Anspruch 10, worin die Aminogruppen des Arginins und/oder der Aminosäure X, insbesondere des Lysins, substituiert sind durch eine der folgenden Gruppierungen:

$C_1$-$C_7$ - Alkyl, $C_6$-$C_{12}$ - Aryl, $C_7$-$C_{20}$ - Alkaryl, $C_7$-$C_{20}$ - Aralkyl, $C_2$-$C_7$ - Alkenyl, $C_2$-$C_7$ - Alkinyl, $C_3$-$C_7$ - Cycloalkyl, $C_3$-$C_7$ -Cycloalkenyl, $C_1$-$C_{18}$ - Alkanoyl, $C_4$-$C_{12}$ -Cycloalkanoyl, $C_7$-$C_{11}$ -Aroyl, $C_6$-$C_{12}$ - Polydroxyalkanoyl, $C_7$-$C_{24}$ - Aralkanoyl,

- $CO(CH_2)_m$-CO-$R^5$, -CO$(CHOH)_n$-CO-$R^5$, mit m = 1-8 und n = 1-12,

wobei $R^5$ OH bzw.deren entsprechende Salze, Ester, und Amide, $N^\alpha$ - Arg - X - Ala - $R^1$ oder $N^\epsilon$ - Lys - (Arg) - Ala - $R^1$ bedeutet.

12. Dimere der Spleninderivate nach den Ansprüchen 10 und 11, die durch Verknüpfung der α-Carboxylgruppe des Alanins bzw. der Aminosäure Z über eine der folgenden Gruppierungen erhalten werden:

-O-$(CH_2)_p$-O-,

-NH-$(CH_2)_p$-NH-,

-O-(CH$_2$-CH$_2$-O-)$_q$-,

-NH-(CH$_2$-CH$_2$-O-)$_q$-CH$_2$-CH$_2$-NH- mit p = 1-18 und q = 1-10 000 .

13. Spleninderivate nach den Ansprüchen 10 bis 12, wobei X Lys und R$^1$ Val-Tyr-OR$^3$ oder Val-Tyr- N-(R$^3$•R$^4$) bedeuten und Arg und/oder Lys und/oder Val-Tyr substituiert sind.

14. Spleninderivate nach den Ansprüchen 10 bis 13, die ausgewählt sind aus der folgenden Gruppe:

(N$^\alpha$-Acyl-Arg) - X - Ala - R$^1$,

Arg - (N$^\epsilon$-Acyl-Lys) - Ala-R$^1$,

(N$^\alpha$-Acyl-Arg) - (N$^\epsilon$-Acyl- Lys) - Ala - R$^1$,

wobei Acyl bevorzugt Acetyl, X bevorzugt Lys und R$^1$ bevorzugt Val-Tyr-OR$^3$ oder Val-Tyr-N (R$^3$ R$^4$) bedeuten, Valin und Tyrosin ggf. substituiert sind.

15. Pharmazeutische Zubereitung mit immunmodulierender Wirkung, gekennzeichnet durch einen Gehalt an mindestens einem Spleninderivat nach einem der Ansprüche 10 bis 14 und einem pharmakologisch unbedenklichen Trägermaterial.

16. Pharmazeutisches Mittel mit immunmodulierender Wirkung gekennzeichnet durch einen Gehalt an mindestens einem Spleninderivat nach einem der Ansprüche 10 bis 14 und einem pharmakologisch unbedenklichen Trägermaterial und/oder Verdünnungsmittel.

17. Verwendung der Spleninderivate nach einem der Ansprüche 10 bis 14 für die Herstellung von pharmazeutischen Zubereitungen zur Normalisierung des Immunsystems, zur Behandlung viraler Infektionen, zur Behandlung nach chemotherapeutischen Maßnahmen, zur Stimulierung des Wachstums und der Reifung von Knochenmarkzellen, zur Therapie von HIV-Infektionen, zur Verhinderung von immunsuppresiven Wirkungen bei chronischer Intoxikationen, wie chronischem Alkoholkonsum und zur Therapie von Autoimmunreaktionen.

18. Verfahren zur Herstellung einer pharmazeutischen Zubereitung, gekennzeichnet dadurch, daß man ein Spleninderivat nach einem der Ansprüche 10 bis 14 mit einem pharmakologisch verträglichen Trägermaterial und/oder Verdünnungsmittel vermischt.

**Europäisches Patentamt**

# EUROPÄISCHER RECHERCHENBERICHT

Nummer der Anmeldung

## EP 90 11 4150

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 067 425 (RICHTER GEDEON VEGYESZETI GYAR R.T.) <br> * Zusammenfassung * Beispiel 1 * <br> --- | 1 | C 07 K 5/08 <br> C 07 K 5/10 <br> C 07 K 7/06 <br> C 07 K 5/02 <br> A 61 K 37/02 |
| Y | EP-A-0 165 033 (ORTHO PHARMACEUTICAL CORPORATION) <br> * Anspruch 1 * <br> --- | 1 | |
| Y | EP-A-0 307 553 (VEB BERLIN-CHEMIE) <br> * Anspruch 1 * <br> ----- | 1 | |

| RECHERCHIERTE SACHGEBIETE (Int. Cl.5) |
|---|
| C 07 K |

Der vorliegende Recherchenbericht wurde für alle Patentansprüche erstellt

| Recherchenort | Abschlußdatum der Recherche | Prüfer |
|---|---|---|
| Den Haag | 14 November 90 | BEVAN S.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument
-------------------------------------------
& : Mitglied der gleichen Patentfamilie, übereinstimmendes Dokument